Europäisches Patentamt

European Patent Office

Office européen des brevets

(11) Publication number: **0 345 726**
**A2**

# EUROPEAN PATENT APPLICATION

(21) Application number: 89110218.8

(22) Date of filing: 06.06.89

(51) Int. Cl.4: **C12N 15/00** , **C07K 7/00** ,
**C12P 21/02** , **C12Q 1/68** ,
**G01N 33/574**

(30) Priority: 07.06.88 US 203434

(43) Date of publication of application:
13.12.89 Bulletin 89/50

(84) Designated Contracting States:
AT BE CH DE ES FR GB GR IT LI LU NL SE

(71) Applicant: INSTITUTE FOR MEDICAL
RESEARCH
2260 Clove Drive
San Jose, Ca 95128(US)

(72) Inventor: Leavitt, John Christopher
411 Maureen Avenue
Palo Alto, CA 94306(US)
Inventor: Lin, Ching-Shwun
3250 Glendale Avenue /A
Menlo Park, CA 94025(US)
Inventor: Aebersold, Ruedi Hans
4638 West 14th Avenue
Vancouver British Columbia V6R 2Y6(CA)

(74) Representative: Barz, Peter, Dr. et al
Patentanwälte Dipl.-Ing. G. Dannenberg Dr.
P. Weinhold, Dr. D. Gudel Dipl.-Ing. S.
Schubert, Dr. P. Barz Siegfriedstrasse 8
D-8000 München 40(DE)

(54) Plastin isoforms and their uses.

(57) A method and reagents are provided for determining whether a human cell is a hemopoietic cell and whether a human tissue cell is in a neoplastic state. Human cells which express only leukocyte-plastin (I-plastin) are hemopoietic cells and human cells which express both I-plastin and tissue-plastin (t-plastin) are neoplastic. The method can be performed using isoform-specific plastin nucleotide probes or isoform-specific anti-plastin antibodies.

EP 0 345 726 A2

## PLASTIN ISOFORMS AND THEIR USES

The present invention relates to methods for characterizing cells and in particular to methods for determining whether a human cell is a hemopoietic cell and whether a human tissue cell is neoplastic.

The importance of oncogenes in the development of human cancer has been amply demonstrated in recent years by the ability of these genes to cause tumorigenic conversion of rodent cells. There can be no doubt that other human genes exist which are not classified as oncogenes per se but which play important roles in the development and progression of cancer. One category of these genes encodes abundant structural proteins such as the actins and tropomyosins. The involvement of these abundant proteins in the neoplastic transformation process is suggested by the well documented observations that isoforms within these structural protein families are consistently modulated in transformation of avian, rodent and human cells. This second category of cancer-related genes is set apart from the so-called "oncogenes" because modulation of these genes in a transformation-sensitive manner is likely to result from regulatory processes activating transcription or translation rather than by direct activation through mutational processes.

An abundant phosphorylated polypeptide, plastin, is frequently expressed in human cancer cells of solid tissue but is not expressed in normal human fibroblasts. The same protein was one of the most abundant constitutively expressed proteins of human white blood cells. Thus, plastin may be associated with transformation of non-hemopoietic cells. Despite the reproducible identification of plastin and studies of its expression and polymorphic character, nothing was known of its molecular identity.

The abundant proteins synthesized in normal and transformed human fibroblasts were examined by means of comparative high resolution two-dimensional polyacrylamide gel electrophoresis. An abundant phosphorylated polypeptide plastin ($M_r$ 68,000, pI 5.3) that is frequently expressed in human cancer cells of solid tissue but that is not expressed in normal human fibroblasts was identified (Leavitt et al., Cell (1982) 28:259-268; Goldstein et al., Cancer Res. (1985a) 45:3256-3261; Leavitt et al., Mol. Cell. Biol. (1986) 6:2721-2726; Leavitt et al., J. Biol. Chem. (1980) 255:1650-1661; Leavitt et al., Carcinogenesis (London) (1982) 3:61-70). When proteins of normal peripheral blood leukocytes were examined, the same protein was one of the most abundant constitutively expressed proteins of white blood cells (Goldstein et al., Cancer Res. - (1985b) 45:5643-5647). Cross-identification of plastin in the transformed fibroblasts and leukocytes was based upon the finding that the protein identified as plastin in mixed proteins of the two cell types comigrated in a two-dimensional gel as a single spot. Furthermore, two separate peptide antibodies specific for the amino acid sequence around residue 244 of actin cross-reacted with plastin from either transformed fibroblasts or lymphocytes in a two-dimensional (2-D) gel western blot (Varma et al., Exp. Cell. Res. (1987) 173:163-173).

Goldman, Merril and colleagues (Goldman et al., Clin. Chem. (1982) 28:1021-1025) observed that plastin was polymorphic in charge among 28 individuals whose leukocyte proteins were examined by 2-D gel electrophoresis. They later referred to this protein as NIMH4 and NC4 (Goldman et al., Am. J. Hum. Genet. (1983) 35:827-837; Goldman et al.,, Am. J. Hum. Genet. (1985) 37:898-911). NIMH4 (or NC4) and I-plastin of fibroblasts were determined to be the same polypeptide (Leavitt et al. (1982), supra). Later, Goldstein et al. also reported that I-plastin was polymorphic in human leukocytes (Goldstein et al. (1985b), supra). Independently, Kondo and Hamaguchi (Kondo et al., Am. J. Hum. Genet. (1986) 37:1106-1111) used the polymorphic character of plastin (LCP1, NIMH4, NC4, or p219.p220) in a gene segregation analysis to link the I-plastin gene to the esterase D and retinoblastoma locus on human chromosome 13.

A method and reagents are provided for determining whether a human cell is a hemopoietic cell and whether a human tissue cell is in a neoplastic state. Human cells which express only leukocyte-plastin (I-plastin) are hemopoietic cells and human cells which express both I-plastin and tissue-plastin (t-plastin) are neoplastic. The method can be performed using isoform-specific plastin nucleotide probes or isoform-specific anti-plastin antibodies.

A method for distinguishing human hemopoietic cells, normal human tissue cells and neoplastic human tissue cells is provided. The method is based on the observation that there are two isoforms of plastin, leukocyte-plastin (I-plastin) and tissue-plastin (t-plastin). Tissue cells express t-plastin, while neoplastic tissue cells additionally express I-plastin. Hemopoietic cells express I-plastin, but neither neoplastic nor normal hemopoietic cells express t-plastin. Therefore a cell that expresses t-plastin is a tissue cell; a cell that expresses only I-plastin is a hemopoietic cell; and a cell that expresses both t-plastin and I-plastin is a neoplastic tissue cell. Isoform-specific nucleic acid probes and isoform-specific anti-plastin antibodies are also provided.

It has now been found that the two isoforms of plastin, t-plastin and I-plastin, have approximately 80% of their amino acids in common and the genes encoding the isoforms have about 60% of their nucleotides in common. The l-plastin isoform is polymorphic. There are at least two species of the t-plastin isoform, which differ by charge. It has not been determined whether these species are encoded by different genes or are encoded by the same gene and differ as the result of post-translational processing. However, a full-length DNA sequence encoding t-plastin hybridizes with mRNA producing each of the t-plastin species under stringent hybridization conditions. Therefore, although the two species of t-plastin may actually be different isoforms of the protein, they will be referred to as the t-plastin isoform for purposes of this application, since each is distinguishable from the l-plastin isoform using the same nucleotide probe or antibody composition.

A DNA fragment of at least about 1700 bp and fewer than about 50 kbp, usually fewer than 30 kbp, comprising a DNA sequence encoding a human plastin isoform or the 5' and 3' flanking non-coding regions is provided. The fragment may be a cDNA sequence comprising the doding and adjacent transcribed regions, usually of not more than about 5 kbp, or a genomic sequence including non-transribed regions of the gene. The isolation of cDNA sequences and genomic DNA sequences encoding l-plastin and t-plastin is described in detail in the Experimental section. Table 2 in the Experimental section provides the complete nucleotide sequences and the deduced amino acid sequences of cDNA fragments encoding t-plastin and l-plastin isolated from a λgt10 cDNA library of transformed human fibroblasts.

The fragment finds use as a probe for detecting other human plastin-encoding genes or closely-related genes in human or other species and for detecting mRNA expressing human plastin. The fragment may also be used to express the encoded plastin isoform by preparing by conventional means an expression construct containing the fragment under the transcriptional and translational control of a promoter. The promoter may be a eukaryotic promoter for expression in a mammalian cell. In cases where one wishes to expand the promoter or produce the peptide isoforms or fragments thereof in a prokaryotic host, the promoter may also be a prokaryotic promoter. Usually a strong promoter will be employed for high level transcription and expression.

The expression construct may be part of a vector capable of stable extrachromosomal maintenance in an appropriate cellular host or may be integrated into host genomes. Normally, markers are provided with the expression construct which allow for selection of a host containing the construct. The marker may be on the same or a different DNA molecule, desirably, the same DNA molecule. In prokaryotic cells, the isoform may serve as a marker or markers such as resistance to a cytotoxic agent, complementation of an auxotrophic host to prototrophy, production of a detectable product etc. will be convenient.

The expression construct can be joined to a replication system recognized by the intended host cell. Various replication systems include viral replication systems such as retroviruses, simian virus, bovine papilloma virus, or the like. In addition, the constuct may be joined to an amplifiable gene, e.g. DHFR gene, so that multiple copies of the fragment may be made. Introduction of the construct into the host will vary depending on the construct and can be acheived by any convenient means. A wide variety of hosts may be employed for expression of the peptides, both prokaryotic and eukaryotic.

Nucleotide sequences of at least about 20 nucleotides and not more than about 50 kbp, usually less than 30 kbp, which include at least about 20 consecutive nucleotides complementary to a DNA sequence encoding a plastin isoform or the adjacent non-coding regions also find use as probes. Usually the nucleotides will be complementary to the transcribed portions of the plastin gene, desirably the coding region, however, the untranscribed regions find use in isolating genomic sequences. Usually the probe will have at least about 50, more usually about 100, generally 500 nt complementary nucleotides. Desirably, when using probes of less than about 500 nt, the probe will share not more than about 60% homology with a DNA sequence encoding the other plastin isoform, usually not more than about 50% homology. In a preferred embodiment, the probe will be complementary to at least a portion of a DNA sequence encoding the N-terminal 20 amino acids, usually the N-terminal 15 amino acids of the plastin isoform. Desirably, the probe will include a sequence encoding the N-terminal 20 amino acids of the plastin isoform. In a preferred embodiment, the probe will include an oligonucleotde sequence in Table 1B. The probes may be used to detect genes encoding plastin or to express at least a portion of the plastin isoform. Usually, however, the probe will be a DNA sequence which finds use to determine whether a cell produces mRNA expressing the isoform.

A substantially pure composition of a plastin isoform may be produced by expressing a DNA fragment of this invention. Peptides corresponding to a portion of the plastin isoform peptide sequence can be produced by recombinant technology or can be chemically synthesized. In a preferred embodiment, a peptide of less than about 100 amino acids comprises at least about 10 consecutive amino acids, more usually at least 15 consecutive amino acids, from the amino acid sequence of a plastin isoform or sequences immunologically cross-reactive therewith. Desirably, the peptide will have at least about 5 consecutive amino acids, usually 10, more usually 15, of the N-terminal about 15 to about 20 amino acids

3

of the plastin isoform sequence. Peptides of less than about 50 amino acids, more usually less than about 30 amino acids, and comprising about 15 to about 20 amino acids of the N-terminal sequence of the plastin isoform may find use to induce isoform-specific anti-plastin antibodies. In a preferred embodiment, the peptide sequence will include at least about 10 consecutive amino acids form the sequence MLDGDRNKDGKISFDEFVYI or MATGDLDQDGRISFDEFIKI.

The peptides may also be used to quantify a peptide isoform as controls or as analyte analogue in competitive inhibition analyses, to determine the specificity of an antibody composition or to purify an antibody composition.

Desirably, peptides used for production of isoform-specific antibodies will share not more than about 60% homology, usually not more than about 50% homology, desirably 40% homology or less, with the other plastin isoform. The first 15 to 20 amino acids of the N-terminus of plastin isoforms share fewer common amino acids than many other portions the isoform sequence of the same length. Thus, the N-terminal sequence of the isoform is conveniently included in the peptides.

An isoform-specific anti-plastin antibody composition reacts with a plastin isoform and exhibits substantially no reaction with the other plastin isoform. The antibody composition desirably has an affinity for the isoform suitable for detection of the isoform on a solid substrate, particularly a Western blot, and exhibits only a backgound level of binding with the other isoform. The antibody affinity required for detection of a plastin isoform using a particular immunoassay method will not differ from that required to detect other polypeptide analytes. The antibody composition may be polyclonal or monoclonal, desirably monoclonal.

Isoform-specific antibodies can be produced by a number of methods. Polyclonal antibodies may be induced by administering an immunogenic composition comprising a peptide of this invention to a host animal. Preparation of immunogenic compositions may vary depending on the host animal and are well known. For example, the peptide may be conjugated to an immunogenic substance such as KLH or BSA or provided in an adjuvant or the like. The induced antibodies can be tested to determine whether the composition is isotype-specific. If a polyclonal antibody composition does not provide the desired specificity, the antibodies can be purified to provide an isoform-specific composition by a variety of conventional methods. For example, when one isoform is used to induce antibodies, the composition can be contacted with the other isoform affixed to a solid substrate to remove those antibodies which bind to the other isoform. Either prior to or following that purification, if desired, the composition can be purified to reduce binding to other substances by contacting the composition with the desired isoform affixed to a solid substrate. Those antibodies which bind to the desired isoform are retained. Purification techniques using peptides or antibodies affixed to a variety of solid substrates such as affinity chromatogaphy materials including Sephadex, Sepharose and the like are well known.

Monoclonal isoform-specific anti-plastin antibodies may also be prepared by conventional methods. A mouse can be injected with an immunogenic composition comprising a peptide of this invention and spleen cells obtained. Those spleen cells can be fused with a fusion partner to prepare hybridomas. Antibodies secreted by the hybridomas can be screened to select a hybridoma wherein the antibodies react with one plastin isoform and exhibit substantially no reaction with the other plastin isoform. Hybridomas that produce antibodies of the desired specificity are cultured by standard techniques. Hybridoma preparation techniques and culture methods are well known and constitute no part of the present invention.

A method of determining whether a cell is hemopoietic comprises determining whether l-plastin, but not t-plastin is present in the cell. Cells that have l-plastin but not t-plastin are hemopoietic. Prior to the present invention cells could be determined to be hemopoietic by a variety of means. For example, cells can be determined to be hemopoietic histologically or by detecting the presence of a variety of markers specific for different types of hemopoietic cells. The present technique is particu larly advantageous in that a marker common to a plurality of types of hemopoietic cells had not previously been found. As described in detail in the Experimental section, it has now been determined that all cells of hemopoietic origin except fully-differentiated red blood cells, exhibit abundant synthesis of l-plastin, and fail to express t-plastin. Therefore, l-plastin is a marker common to all cells of hemopoietic origin. Further, all cells that express l-plastin but fail to express t-plastin are hemopoietic cells.

A method for determining whether a tissue cell is in a neoplastic state comprises determining whether l-plastin is present in the cell. Tissue cells which express l-plastin are neoplastic. Although the cell can be determined to be a non-hemopoietic cell by any of a number of methods, conveniently, the cell will be determined to be a tissue cell by detecting the presence of t-plastin. A cell which expresses both t-and l-plastin is a neoplastic tissue cell. Of course, if a cell expresses t-plastin but does not express l-plastin, the cell may be neoplastic. That is, a positive result (presence of both t- and l-plastin) indicates that a cell is neoplastic, but a negative result is not conclusive.

To determine whether a cell expresses l- or t-plastin, an isoform-specific plastin probe or an isoform-specific anti-plastin antibody can be used to detect the presence of mRNA or plastin peptide isoform, respectively. When using a probe, the probe will be hydridized to mRNA from the cell under stringent hybridization conditions. An exemplary method of detecting mRNA encoding a plastin isoform by a Northern blot analysis is described in the Experimental section.

Detecting mRNA is conveniently performed on a cell which has been cultured to provide a number of identical cells sufficient for mRNA analysis. However, in neoplastic tissue cells, the amount of l-plastin mRNA present in a cell comprises about 0.1% to about 200% of the amount of t-plastin mRNA. Therefore, the sample being analyzed has been determined to contain only a small percentage of hemopoietic cells, desirably less than 0.001% by weight, the presence of l-plastin mRNA in a substantial percentage of the tissue cells, usually at least about 0.1%, desirably 1.0% or more, can be distinguished from l-plastin mRNA in the contaminating hemopoietic cells.

Alternatively, the presence of t- or l-plastin can be detected using isoform-specific anti-plastin antibodies. A variety of methods of detecting polypeptides with specific antibodies are well known. Since plastin comprises a major component of the cell cytosol, determination of which isoforms are present in particular cells is conveniently performed by preparing the cells for staining by standard histologic techniques such as embedding formalin-fixed cells in paraffin. The isoform specific antibodies can be conjugated to distinguishable labels such as rhodamine and fluorescein and used simultaneously to stain the cells. Such methods unambiguously indicate the presence of both isoforms within a cell.

However, similar techniques may be sufficient in particular circumstances. For example, samples could be sequentially stained with each of the isoform-specific antibodies. When a statistically larger percentage of cells stain with anti-l-plastin than do not stain with anti-t-plastin, there are cells in the sample that contain both l- and t-plastin. That or similar methods may also find use with samples substantially free from hemopoietic cells or samples having a significant percentage of neoplastic cells.

Additionally, quantitative measurements of plastin isoforms in various bodily fluids such as serum and human milk may be indicative of disease states. For example, elevated levels of l-plastin in serum may be indicative of lytic infections of lymphocytes present in diseases such as AIDS. A preferred assay is a Western blot which definitively demonstrates that the correct peptide has been detected through the combination of the use of gel electrophoresis and immunoassay analysis. However, other immunoassay methods may also find use. Numerous quantitative immunoassay methods for detecting a peptide in a bodily fluid are known. An assay method has the following elements. The method comprises combining the sample with the isoform-specific antibody and detecting the presence of isoform-specific antibody-peptide complex as indicative of the presence of the peptide. in the sample. The particular manner in which the peptide is detected is not significant for the purpose of this invention so long as the method provides the desired degree of sensitivity and reliability.

A number of different types of immunoassays are well known using a variety of protocols and labels. The assay conditions and reagents may be any of a variety found in the prior art. The assay may be heterogeneous or homogeneous, usually heterogeneous, conveniently a sandwich assay.

The assay will usually employ solid phase-affixed isoform specific anti-plastin antibodies. The antibodies may be polyclonal or monoclonal, usually monoclonal. The solid phase-affixed antibodies are combined with the sample. Binding between the antibodies and sample can be determined in a number of ways. Complex formation can be determined by use of soluble antibodies specific for the isoform to be detected. The antibodies can be labeled directly or can be detected using labeled second antibodies specific for the species of the soluble antibodies. Various labels include radionuclides, enzymes, fluorescers or the like. Conveniently, the assay will be an enzyme-linked immunosorbant assay (ELISA) in which monoclonal antibodies specific for different epitopes of the plastin isoform are used as the solid phase-affixed and enzyme labeled, soluble antibodies.

Alternatively, the assay may be based on competitive inhibition, where plastin in the sample competes with a known amount of a plastin isoform for a predetermined amount of isoform-specific anti-plastin antibody. For example, any of the plastin isoform present in the sample can compete with a known amount of the labeled plastin isoform or isoform analogue for antibody binding sites. The amount of labeled isoform affixed to the solid phase or remaining in solution can be determined.

The following examples are offered by way of illustration and not by way of limitation.

## EXPERIMENTAL

## Example 1

Isolation and Sequencing of cDNA Encoding Plastin Isoforms

Two isolation techniques which were developed for protein microsequencing (Aebersold et al.,J. Biol. Chem. (1986) 261:4229-4238; Aebersold et al., Methods in protein sequence analysis, K. Walsh, ed., Human Press. Clifton, NJ (1987a), p. 277-294; Aebersold et al., Proc. Natl. Acad. Sci. USA (1987b) 84:6970-6974) were used to obtain sequence information from plastin purified from 2-dimensional gels. For N-terminal sequence analysis, proteins were electroblotted from analytical two-dimensional gels onto chemically-modified glass fiber filter paper and detected by fluorescent staining. Plastin spots were cut out and inserted into the sequenator cartridge for direct sequence analysis (Aebersold et al. (1986), supra; Aebersold et al. (1987a), supra). The amino acid sequence analysis was fperformed on an automated Caltech gas-phase sequenator (Hewick et al., J. Biol. Chem (1981) 256:7990-7997).

In repeated attempts, an N-terminal sequence from T-lymphocyte plastin was not obtained, although proteins expressed at comparable levels and simultaneously isolated from the same two-dimensional gels were readily sequenced (Aebersold et al. (1987a), supra). This suggested that plastin was blocked at the amino terminal end. Using a newly-developed procedure (Aebersold et al. (1987b), supra), internal protein sequence information of plastin was obtained.

Proteins in a total cell lysate of CEM lymphoblastoid cells were separated by two-dimensional gel electrophoresis and electroblotted onto nitrocellulose. Plastin containing spots were excised and enzymatically cleaved on the nitrocellulose matrix (Aebersold et al. (1987b), supra). The resulting peptides were separated by narrow-bore reversed-phase HPLC and individual peptide fragments were sequenced in a modified Caltech gas-phase sequenator (Kent et al., Biotech. (1987) 5:314-321). Four peptide sequences that were unambiguously identified are listed in Table 1.

TABLE 1

| A. Four Unambiguous Plastin Peptide Sequences |
| --- |
| ...Glu-Val-Ile-Pro-Met-Asn-Pro-Asn-Ser,or Thr-Asn-Asp-blank-Phe-blank-Ala-Val... |
| ...Thr-Ile-Gln-Glu-Asn-Leu-Asn-Leu-Ala-Leu-Asn-Ser-Ala-Ser-Blank-Ile-Gly... |
| ...Val-Asn-Asp-Asp-Ile-Ile-Val-Asn-Tyr-Val-Asn-Glu... |
| ...Ile-Ser-Thr-Ser-Leu-blank-Val-Leu-Asp-Leu-Ile-Asp... |

| B. Synthesis of a Degenerate Oligonucleotide Probe for Plastin | | | |
| --- | --- | --- | --- |
| Amino Acid Position | Oligopeptide Sequence | Oligonucleotide Probe (Antisense) | Corresponding 1 -Plastin Nucleotide Sequence (Sense) |
| | | 3′ end | |
| 1 | Val | C | G |
| | | A | T |
| | | CG | C |
| 2 | Asn | T | A |
| | | T | A |
| | | AG | T |
| 3 | Asp | C | G |
| | | T | A |
| | | AG | T |
| 4 | Asp | C | G |
| | | T | A |
| | | AG | C |
| 5 | Ile | T | A |
| | | A | T |
| | | I | T |
| 6 | Ile | T | A |
| | | A | T |
| | | I | T |
| 7 | Val | C | G |
| | | A | T |

A degenerate oligonucleotide 20-mer corresponding to one of the four oligopeptides (shown in Table 1) was synthesized and used to screen a λgt10 cDNA library of transformed (HuT-14) human fibroblasts (Lin et al., J. Mol. Cell. Biol. (1988) 8:160-168). From approximately 10,000 recombinants, the probe selected a single clone, P4.

Cellular RNAs were prepared by the guanidine hydrochloride method (as previously described in Gunning et al., J. Mol. Evol.(1984) 20:202-214). Five microgram of each RNA was electrophoresed in a 1% agarose gel containing 50 mM morpholinepropanesulfonic acid (pH 7), 1 mM EDTA, and 2.2 M formaldehyde; blotted onto nitrocellulose; and hybridized with nick-translated probes (Rigby et al., J. Mol. Biol. - (1977) 113:237-251). Hybridization proceeded overnight at 65°C in 4x SSC (1x SSC is 150 mM NaCl and 15 mM sodium citrate, pH 7), 5x Denhardt's solution (Denhardt, Biochem. Biophys. Res. Comm. (1966) 23:641-646), 50 mM phosphate buffer (pH 7), 10% (w/v) dextran sulfate, and 2 X 10⁶ cpm/ml probe. Washing was carried out twice in 1x SSC and 0.1% (w/v) sodium dodecyl sulfate (SDS) at room temperature for 5 min each, and twice in 0.5x SSC and 0.1% SDS at 65°C for 30 min each. Two identical Northern blots were hybridized using Northern analysis with $^{32}$P-labeled cDNAs of P4 and P107, respectively. Each blot contained cellular RNAs of CEM, KD, and HuT-14. Size markers were 28S and 18S ribosomal RNAs (5 kb and 2 kb, respectively).

The cDNA insert of P4 was 4.3 kilobases (kb) in length. (This large cDNA was later determined to be comprised of two unrelated cDNA fragments of 1.0 kb and 3.3 kb that were ligated during the construction of the library.) A 2-kb HindIII fragment within the 3.3-kb cDNA insert of P4 hybridized to an mRNA of 3.4 kilobases that was more abundant in HuT-14 than in normal KD fibroblasts, and not detectable in CEM T-lymphocytes.

cDNA was cloned into M13mp9 (Messing, J. Methods in Enzymol. (1983) 101:20-78). Progressive deletion clones were prepared by the method described by Dale et al., Plasmid (1985) 13:31-40. Sequencing was done by the method described by Sanger et al., Proc. Natl. Acad. Sci. USA (1977) 74:5463-5467. Table 2 illustrates the coding sequences and deduced amino acid sequences of P4 cDNA (top, t-plastin) and P107 cDNA (bottom, I-plastin. Identical nucleotides between the two sequences are indicated by double dots. As shown in the table, the cDNA sequences share about 60% homology, while the peptide sequences share about 80% homology.

```
          M   L   D   G   D   R   N   K   D   G   K   I   S   F   D   E   F   V   Y   I   F   Q   E 23
   P4  ─⟩ATGCTGGATGGTGACAGGAATAAAGATGGGAAAATAAGTTTTGACGAATTTGTTTATATTTTTCAAGAG
         :::        :::::     : :   ::::::::  :   :: ::  :::::  ::  ::: :    :  :::::  :: :
  P107 ─⟩ATGGCTACAGGTGATCTGGACCAAGATGGAAGGATCAGCTTTGATGAGTTTATCAAGATTTTCCATGGC
          M   A   T   G   D   L   D   Q   D   G   R   I   S   F   D   E   F   I   K   I   F   H   G

          V   K   S   D   I   A   K   T   F   R   K   A   I   N   R   K   E   G   I   C   A   L 46
         GTAAAAAGTAGTGATATTGCCAAGACCTTCCGCAAAGCAATCAACAGGAAAGAAGGTATTTGTGCTCTG
         :::::::  :   ::: :::::::::::::::  :  ::::::::::::  :  ::: :::::  :::::::::  :
         CTAAAAAGCACAGATGTTGCCAAGACCTTTAGAAAAGCAATCAATAAGAAGGAAGGGATTTGTGCAATC
          L   K   S   T   D   V   A   K   T   F   R   K   A   I   N   K   K   E   G   I   C   A   I

          G   G   T   S   E   L   S   S   E   G   T   Q   H   S   Y   S   E   E   E   K   Y   A   F 69
         GGTGGAACTTCAGAGTTGTCCAGCGAAGGAACACAGCATTCTTACTCAGAGGAAGAAAAATATGCTTTT
         :::::  :::::::::  :::  ::::   ::  ::  ::  ::  :: ::  ::::::::::::  :::::  :::::  ::
         GGTGGTACTTCAGAGCAGTCTAGCGTTGGCACCCAACACTCCTATTCAGAGGAAGAAAAGTATGCCTTT
          G   G   T   S   E   Q   S   S   V   G   T   Q   H   S   Y   S   E   E   E   K   Y   A   F

          V   N   W   I   N   K   A   L   E   N   D   P   D   C   R   H   V   I   P   M   N   P   N 92
         GTTAACTGGATAAACAAAGCTTTGGAAAATGATCCTGATTGTAGACATGTTATACCAATGAACCCTAAC
         ::  ::::::::::::::::::::::     ::::::::::::::::  : ::::: :: :  ::::::::::  :::
         GTCAACTGGATAAACAAAGCCCTGGAAAATGATCCTGATTGTCGGCATGTCATCCCAATGAACCCAAAC
          V   N   W   I   N   K   A   L   E   N   D   P   D   C   R   H   V   I   P   M   N   P   N
                                           peptide# 1 ▶ E   V   I   P   M   N   P   N
          T   D   D   L   F   K   A   V   G   D   G   I   V   L   C   K   M   I   N   L   S   V   P 115
         ACCGATGACCTGTTCAAAGCTGTTGGTGATGGAATTGTGCTTTGTAAAATGATTAACCTTTCAGTTCCT
         ::   ::::::  ::::::: :::::  ::::: :::::  :::::::::::::::::::::  :::::  :::::   ::
         ACGAATGATCTCTTTAATGCTGTTGGAGATGGCATTGTCCTTTGTAAAATGATCAACCTGTCAGTGCCA
          T   N   D   L   F   N   A   V   G   D   G   I   V   L   C   K   M   I   N   L   S   V   P
         SorT N   D   -   F   -   A   V
          D   T   I   D   E   R   A   I   N   K   K   K   L   T   P   F   I   I   Q   E   N   L   N 138
         GATACCATTGATGAAAGAGCAATCAACAAGAAGAAACTTACACCCTTCATCATTCAGGAAAACTTGAAC
         ::  ::  :::::::::::::::  ::::::::::::  :::::  ::  ::  ::  ::::  :::::::::::::::  :::::
         GACACAATTGATGAAAGAACAATCAACAAAAAGAAGCTAACCCCCTTTCACCATTCAGGAAAATCTGAAC
          D   T   I   D   E   R   T   I   N   K   K   K   L   T   P   F   T   I   Q   E   N   L   N
                                      peptide # 2 ▶ T   I   Q   E   N   L   N
```

8

```
        L   A   L   N   S   A   S   A   I   G   C   H   V   V   N   I   G   A   E   D   L   R   A 161
        TTGGCACTGAACTCTGCTTCTGCCATTGGGTGTCATGTTGTGAACATTGGTGCAGAAGATTTGAGGGCT
        : : : : :   : : : : : : : : :   : :     : : : : :   : :     : :     : :     : :       : : :     : :
        TTGGCTCTGAACTCTGCCTCAGCCATCGGGTGCCATGTGGTCAACATAGGGGCTGAGGACCTGAAGGAG
        L   A   L   N   S   A   S   A   I   G   C   H   V   V   N   I   G   A   E   D   L   K   E
        L   A   L   N   S   A   S   —   I   G
       _____

        G   K   P   H   L   V   L   G   L   L   W   Q   I   I   K   I   G   L   F   A   D   I   E 184
        GGGAAACCTCATCTGGTTTTGGGACTGCTTTGGCAGATCATTAAGATCGGTTTGTTCGCTGACATTGAA
        : : : : :   : : :   : : : : : : :   : : : : : : :   : :   : : : : :     : : : : :   : : : : : : : : : : :
        GGGAAGCCTTATCTGGTCCTGGGACTTCTGTGGCAAGTCATCAAGATTGGGTTGTTTGCTGACATTGAA
        G   K   P   Y   L   V   L   G   L   L   W   Q   V   I   K   I   G   L   F   A   D   I   E

                                                    peptide # 3 ▶ A   D   I   E
                                                              _____
        L   S   R   N   E   A   L   A   A   L   L   R   D   G   E   T   L   E   E   L   M   K   L 207
        TTAAGCAGGAATGAAGCCTTGGCTGCTTTACTCCGAGATGGTGAGACTTTGGAGGAACTTATGAAATTG
        :   : : : : :   : : : : : : : :   : :     : : : :   :     :     : : : :   : : : : : : :         : : : : : : :   :   : : : : : :   :
        CTCAGCAGAAATGAAGCTCTGATTGCTCTTTTGAGAGAAGGTGAGAGCCTGGAGGATTTGATGAAACTC
        L   S   R   N   E   A   L   I   A   L   L   R   E   G   E   S   L   E   D   L   M   K   L
        L
       _
        I

        S   P   E   E   L   L   L   R   W   A   N   F   H   L   E   N   S   G   W   Q   K   I   N 230
        TCTCCAGAAGAGCTTCTGCTTAGATGGGCAAACTTTCATTTGGAAAAACTCGGGCTGGCAAAAAATTAAC
        :   : :   : : : : : : :     : : : :   : :   : : : : :   : :   :   : : : : : : :     :   : : : : :     :   : : : : : :       :
        TCCCCTGAAGAGCTCTTGCTGAGGTGGGCTAATTACCACCTGGAAAATGCAGGCTGCAACAAAATTGGC
        S   P   E   E   L   L   L   R   W   A   N   Y   H   L   E   N   A   G   C   N   K   I   G

        N   F   S   A   D   I   K   D   S   K   A   Y   F   H   L   L   N   Q   I   A   P   K   G 253
        AACTTTAGTGCTGACATCAAGGATTCCAAAGCCTATTTCCATCTTCTCAATCAAATCGCACCAAAAGGA
        : : : : :   : : :   : : : : : : : : : : : : :   : :   : : : : :   : : : :   : : :   : :   :   :   : :     :   : :   : : : : : : : : :
        AACTTCAGTACTGACATCAAGGACTCAAAAGCTTATTACCACCTGCTTGAGCAGGTGGCTCCAAAAGGA
        N   F   S   T   D   I   K   D   S   K   A   Y   Y   H   L   L   E   Q   V   A   P   K   G
                                                       •

        Q   K   E   G   E   P   R   I   D   I   N   M   S   G   F   N   E   T   D   D   L   K   R 276
        CAAAAGGAAGGTGAACCACGGATAGATATTAACATGTCAGGTTTCAATGAAACAGATGATTTGAAGAGA
        :   :   : : : : : : :     : :         :   :   : : : :   : : : : : : :         : :   :   : : : : :   :     : : : :
        GATGAAGAAGGTGTTCCTGCTGTTGTTATTGACATGTCAGGACTGCGGGAGAAGGATGACATCCAGAGG
        D   E   E   G   V   P   A   V   V   I   D   M   S   G   L   R   E   K   D   D   I   Q   R

        A   E   S   M   L   Q   Q   A   D   K   L   G   C   R   Q   F   V   T   P   A   D   V   V 299
        GCTGAGAGTATGCTTCAACAAGCAGATAAATTAGGTTGCAGACAGTTTGTTACCCCTGCTGATGTTGTC
        : :   : :     :   : : : : :   : :   : :   : :   : :   :         :   : :   : : :   :   : : : : : : : :   : :     :     : : : : : : : : :
        GCAGAATGCATGCTGCAGCAGGCGGAGAGGCTGGGCTGCCGGCAGTTTGTCACAGCCACAGATGTTGTC
        A   E   C   M   L   Q   Q   A   E   R   L   G   C   R   Q   F   V   T   A   D   V   V

        S   G   N   P   K   L   N   L   A   F   V   A   N   L   F   N   K   Y   P   A   L   T   K 322
        AGTGGAAACCCCAAACTCAACTTAGCTTTCGTGGCTAACCTGTTTAATAAATACCCAGCACTAACTAAG
        :   :   : : : : : :   : : : : :   : : : : :       : : :   : : : : :   : : : : :   :   : : : : : :   : :   : :             : :
        CGAGGGAACCCCAAGTTGAACTTGGCTTTTATTGCCAACCTCTTTAACAGATACCCTGCCCTGCACAAA
        R   G   N   P   K   L   N   L   A   F   I   A   N   L   F   N   R   Y   P   A   L   H   K

        P   E   N   Q   D   I   D   W   T   L   L   E   G   E   T   R   E   E   R   T   F   R   N 345
        CCAGAGAACCAGGATATTGACTGGACTCTATTAGAAGGAGAAACTCGTGAAGAAAGAACCTTCCGTAAC
        : : : : : : : : : : : :     : : : : : : :         :   : : : : :   : :   : :     :   : : : : :     :   : :   : :         : : :
        CCAGAGAACCAGGACATTGACTGGGGGGGCTCTTGAAGGTGAGACGAGAGAAGAGCGGACATTTAGGAAC
        P   E   N   Q   D   I   D   W   G   A   L   E   G   E   T   R   E   E   R   T   F   R   N
```

```
 W  M  N  S  L  G  V  N  P  H  V  N  H  L  Y  A  D  L  Q  D  A  L  V 368
TGGATGAACTCTCTTGGTGTCAATCCTCACGTAAACCATCTCTATGCTGACCTGCAAGATGCCCTGGTA
:::::::::::::: :: ::::: :: ::::: ::  :::: : :::: :  ::::: :
TGGATGAACTCCCTGGGTGTTAACCCTCGAGTCAATCATTTGTACAGTGACTTATCAGATGCCCTGGTC
 W  M  N  S  L  G  V  N  P  R  V  N  H  L  Y  S  D  L  S  D  A  L  V
                 peptide # 4 ▶ V  N  -  L  Y  -  D  L

 I  L  Q  L  Y  E  R  I  K  V  P  V  D  W  S  K  V  N  K  P  P  Y  P 391
ATCTTACAGTTATATGAACGAATTAAAGTTCCTGTTGACTGGAGTAAGGTTAATAAACCTCCATACCCG
:::::: ::: : :::::::  :: ::::::::::::::::::: :  :::: : ::::: ::::::::
ATCTTCCAGCTCTATGAAAAGATCAAAGTTCCTGTTGACTGGAACAGAGTAAACAAACCGCCATACCCC
 I  F  Q  L  Y  E  K  I  K  V  P  V  D  W  N  R  V  N  K  P  P  Y  P

 K  L  G  A  N  M  K  K  L  E  N  C  N  Y  A  V  E  L  G  K  H  P  A 414
AAACTGGGAGCCAACATGAAAAAGCTAGAAAACTGCAACTATGCTGTTGAATTAGGGAAGCATCCTGCT
::::::::::::  ::: ::::::  ::::: :: :: ::::: :: :: ::::: :::::: ::
AAACTGGGAGGCAATATGAAGAAGCTTGAGAATTGTAACTACGCGGTAGAATTGGGGAAGAATCAAGCG
 K  L  G  G  N  M  K  K  L  E  N  C  N  Y  A  V  E  L  G  K  N  Q  A

 K  F  S  L  V  G  I  G  G  Q  D  L  N  D  G  N  Q  T  L  T  L  A  L 437
AAATTCTCCCTGGTTGGCATTGGAGGGCAAGACCTGAATGATGGGAACCAAACCCTGACTTTAGCTTTA
:: :::::::::::::::::::::: :: :: ::::: :: ::::  :: :::::  :  : :: ::
AAGTTCTCCCTGGTTGGCATCGGTGGACAAGATCTCAATGAAGGAAACCGCACTCTCACACTGGCCTTG
 K  F  S  L  V  G  I  G  G  Q  D  L  N  E  G  N  R  T  L  T  L  A  L
peptide#5 ▶ F  S  L  V  G  I  G  G  Q  D  L  N

 V  W  Q  L  M  R  R  Y  T  L  N  V  L  E  D  L  G  D  G  Q  K  A  N 460
GTCTGGCAGCTGATGAGAAGATATACCCTCAATGTCCTGGAAGATCTTGGAGATGGTCAGAAAGCCAAT
:  ::::::::::  ::::::::  ::::: :: ::: :::: ::::: ::::: :  : ::::
ATTTGGCAGCTAATGAGAAGGTATACACTGAATATCCTCGAAGAAATTGGTGGTGGCCAGAAGGTCAAT
 I  W  Q  L  M  R  R  Y  T  L  N  I  L  E  E  I  G  G  G  Q  K  V  N
                                              peptide # 6 ▶ V  N

 D  D  I  I  V  N  W  V  N  R  T  L  S  E  A  G  K  S  T  S  I  Q  S 483
GACGACATCATTGTGAACTGGGTGAACAGAACGTTGAGTGAAGCTGGAAAATCAACTTCCATTCAGAGT
:: ::::: :::::: :::::::::::::   ::: ::::: :  : ::::: ::::
GATGACATTATTGTCAACTGGGTGAATGAAACATTGAGGGAAGCAGAGAAAAGTTCATCCATCTCTAGT
 D  D  I  I  V  N  W  V  N  E  T  L  R  E  A  E  K  S  S  S  I  S  S
 D  D  I  I  V  N  W  V  N  E

 F  K  D  K  T  I  S  S  S  L  A  V  V  D  L  I  D  A  I  Q  P  G  C 506
TTTAAGGACAAGACGATCAGCTCCAGTTTGGCAGTTGTGGATTTAATTGATGCCATCCAGCCAGGCTGT
:: ::::::: :: ::: ::  : ::: :: :  :::: : ::: : :::::::: ::::::
TTCAAGGACCCGAAGATTAGTACAAGTCTGCCTGTTCTGGACCTCATCGATGCCATCCAACCAGGTTCC
 F  K  D  P  K  I  S  T  S  L  P  V  L  D  L  I  D  A  I  Q  P  G  S
         peptide# 7 ▶ I  S  T  S  L  -  V  L  D  L  I  D

 I  N  Y  D  L  V  K  S  G  H  L  T  E  D  D  K  H  N  N  A  K  Y  A 529
ATAAACTATGACCTTGTGAAGAGTGGCAATCTAACAGAAGATGACAAGCACAATAATGCCAAGTATGCA
:: :::::::::::: ::::::  :  ::::::: :: : ::: :: : ::: :::::
ATTAACTATGACCTTCTGAAGACAGAAAATCTGAATGATGATGAGAAACTCAACAATGCAAAATATGCC
 I  N  Y  D  L  L  K  T  E  N  L  N  D  D  E  K  L  N  N  A  K  Y  A

 V  S  M  A  R  R  I  G  A  R  V  Y  A  L  P  E  D  L  V  E  V  K  P 552
GTGTCAATGGCTAGAAGAATCGGAGCCAGAGTGTATGCTCTCCCTGAAGACCTTGTGGAAGTAAAGCCC
: :: ::::::  ::: ::: :::::  :::::::::::: :: ::  ::::::::  :: ::::: ::  :::
ATCTCTATGGCCCGAAAAATTGGAGCAAGAGTGTATGCCCTGCCAGAAGACCTGGTTGAAGTGAACCCC
 I  S  M  A  R  K  I  G  A  R  V  Y  A  L  P  E  D  L  V  E  V  N  P
               peptide # 8 ▶ V  Y  A  L  P  E  D  L  V  E  V

 K  M  V  M  T  V  F  A  C  L  M  G  R  G  M  K  R  V  . 570
AAGATGGTCATGACTGTGTTTGCATGTTTGATGGGCAGGGGAATGAAGAGAGTGTAA
:: :::::::::::: :::::::: ::  : :::::: :  ::::::::::: :::: :
AAAATGGTCATGACCGTGTTTGCCTGCCTCATGGGGAAAGGAATGAAGAGGGTGTGA
 K  M  V  M  T  V  F  A  C  L  M  G  K  G  M  K  R  V  .
```

When the cDNA was sequenced, four of the oligopeptide sequences that had been unambiguously identified by direct amino acid sequence determination were found (Table 2). The four sequences, peptides No. 1, 2, 6 and 7, starting at amino acid residues 85, 132, 458 and 489, respectively, were unambiguously identified. The other four sequences (peptides No. 3, 4, 5 and 8) were deduced from mixed sequences using the completed cDNA sequence.

There were, however, six differences between the protein sequence (residues 85, 94, 132, 459, 491, 496) derived from the lymphocyte plastin protein sequence and the reverse-translated cDNA sequence of the cDNA isolated from transformed fibroblasts (Table 2B). These differences indicated that a different but closely related cDNA had been cloned. To identify the protein translation product of the cloned P4 cDNA, the entire 4.3-kb cDNA insert was used to select for mRNAs by hybridization from PolyA⁺RNA of HuT-14. Then subsequently, the selected mRNAs were translated in vitro.

Total cellular RNA was prepared by the guanidine hydrochloride method, as described previously

10

(Gunning et al., supra). PolyA[+] RNA was prepared by oligo(dT)-cellulose chromatography (Aviv et al., Proc. Natl. Acad. Sci. USA (1972) 69:1408-1412). The purified cDNA fragment was bound to nitrocellulose paper essentially as described by Parnes et al., Proc. Natl. Acad. Sci. USA (1981) 78:2253-2257, except that the DNA solution was not subjected to boiling and was added to nitrocellulose paper with the aid of a Minifold (Schleicher & Schuell, Inc., Kenne, NH). Hybridization to mRNA and elution of hybridized mRNA were done as described by Maniatis et al., Molecular cloning, a laboratory manual, Cold Spring Harbor Lab., Cold Spring Harbor, NY (1982), except that tRNA was not included in the hybridization solution or during the elution of mRNA. The eluted mRNA was precipitated in the presence of calf liver tRNA (15 Mg/ml) in 70% ethanol. The mRNA was suspended in water and subjected to in vitro translation. All in vitro translation experiments were carried out in a rabbit reticulocyte system purchased from New England Nuclear Corp. (Boston. MA). The translated products were then electrophoresed in a two-dimensional polyacrylamide gel and visualized by autoradiography as described previously (Lin et al., supra). The method of 2-D gel electrophoresis and translation of mRNAs selected by hybridization to cDNAs has been described previously (Lin et al., supra). The samples in each gel were as follows.

A. CEM T-lymphoblastoid total cell (ATCC No. CCL119) unfractionated proteins stained with silver (Merril et al., Electrophoresis (1982) 317-23);

B. KD untransformed human fibroblasts (Leavitt et al. (1986) supra), total cell unfractionated proteins labeled with [35S] methionine (Leavitt et al., J. Biol. Chem. (1980a) 255:1650-1661);

C. HuT-12 transformed human fibroblasts (Leavitt et al. (1986), supra, prepared as in B;

D. HuT-14 transformed human fibroblasts (Leavitt et al. (1986), supra), prepared as in B;

E. normal human monocyte total unfractionated proteins labeled with [35S] methionine (Goldstein et al. (1985b) supra);

F. in vitro translated [35S] methionine labeled polypeptides of total PolyA + mRNA isolated from HuT-14 transformed fibroblasts (Lin et al., supra)

G. in vitro translated polypeptides of PolyA + mRNA from HuT-14 cells used in F and selected by hybridization to the p4 cDNA;

H. in vitro translated polypeptides of PolyA + mRNA from HuT-14 cells used in F and selected by hybridization to the p107 cDNA;

I. in vitro translation (control) as in H, except that no PolyA + mRNA was added to the translation reaction;

J. HuT-12 proteins prepared as in B;

K. HuT-12 proteins prepared as in B, mixed with in vitro translated polypeptides prepared as in H.


Polypeptide X, identified as a 70 kd heat shock response polypeptide in fibroblasts and leukocytes, served as a 2-D gel marker for the more acidic isoform of t-plastin in Gels C and E since it has the same pI and exhibits a slightly higher $M_r$.

The most prominent translation products were two proteins (t-plastins) migrating to more basic isoelectric points than the polypeptide recognized as plastin in the 2-D gel (Gels F, G). These two proteins were abundantly synthesized in both normal and transformed human fibroblasts (Gels B, C), but were undetectable in white blood cells (Gels A and E). As these two proteins were apparently similar to plastin, the 2-kb HindIII fragment of P4 cDNA was used as a probe to rescreen the HuT-14 cDNA library to find related cDNAs.

Several clones were identified by this rescreening. One of them, P107, had the largest insert cDNA (3.7 kb) and was chosen for further examination. In Northern analysis as described above, P107 hybridized to an mRNA of 3.7 kilobases that were more abundant in CEM lymphocytes, less abundant in transformed HuT-14 fibroblasts. and not detectable in untransformed KD fibroblasts. This was the predicted pattern for plastin mRNA expression in these three cell types (Goldstein et al. (1985b), supra). Translation of HuT-14 mRNAs selected by this clone yielded a single polypeptide species that appeared to be identical to plastin in a 2-D gel (Gel H). This in vitro synthesized polypeptide was determined to be electrophoretically identical to I-plastin by mixing the in vitro translation sample in Gel H with labeled unfractionated HuT-12 cellular proteins (Leavitt et al. (1986), supra) shown in Gel J. This in vitro translated I-plastin, which was in excess of the endogenous HuT-12 I-plastin, was superimposed upon the endogenous I-plastin following 2-D gel electrophoresis (Gel K). It was therefore concluded that P107 is the true cDNA clone of I-plastin, whereas P4 cDNA encoded a separate polypeptide isoform, t-plastin, closely related to I-plastin.

DNA sequencing of P107 cDNA revealed a coding sequence closely related to that of the initially characterized P4 clone (Table 2). This P107 cDNA sequence contained the amino acid residues determined by protein sequence analysis of plastin isolated from CEM lymphocytes in the positions where discrepancies had existed between protein sequence and P4 cDNA sequence. Each coding sequence encoded a polypeptide of 570 amino acids with molecular weight of 64 kilodaltons. This value (64kd) is slightly different from the observed $M_r$ value of l-plastin and the two t-plastin polypeptides ($M_r68,000$) in a 2-D gel. Post-transitional modification of the polypeptides, such as glycosylation, may account for this discrepancy.

The predicted amino acid composition obtained for plastin by computerized microdensitometry was consistent with the amino acid composition determined from these DNA sequences (Goldstein et al. (1985b), supra). That l-plastin is expressed in leukocytes and in transformed fibroblasts (HuT-14) but not in normal fibroblasts is now confirmed because the oligopeptide sequences were derived from l-plastin of lymphocytes and the l-plastin cDNA clone was isolated from transformed HuT-14 fibroblasts. The t-plastin isoforms, on the other hand, are expressed in both normal and transformed fibroblasts, but not in leukocytes.


Example 2


Differential Expression of Plastin Isoforms in Human Cell Transformation

A panel of cultured human cell strains was surveyed with Northern analysis for l-plastin (Blot A) and t-plastin mRNA (Blot B). The methods used were as described for Table 1. Two identical Northern blots, A and B, were hybridized with P32-labeled cDNAs of P107, and P4, respectively, to analyze plastin expression. Each blot contained cellular RNAs of the following cell strains.

Lane 1, KD, untransformed human fibroblasts (Leavitt et al., (1986), supra);

Lane 2, R17, untransformed human fibroblasts provided by Thomas Hassel, University of North Carolina;

Lane 3, HuT-12, transformed (neoplastic) human fibroblasts (Leavitt et al., (1986), supra);

Lane 4, HuT-14, transformed, tumorigenic human fibroblasts (Leavitt et al., supra);

Lane 5, HuT-14T, transformed, tumorigenic human fibroblasts (Leavitt et al. (1986), supra);

Lane 6, HT1080, human fibrosarcoma (neoplastic) (Goldstein et al., (1985b) supra);

Lane 7, Sarcoma-2, a neoplastic cell line derived from a human lieomyosarcoma and provided by Professor George Milo, Ohio State University, Columbus, Ohio;

Lane 8, HOS, osteosarcoma (Goldstein, et al. (1985b), supra);

Lane 9, CEM, human T-cell leukemia (Aebersold et al. (1987b), supra);

Lane 10, AG1484 B-lymphoblast strain from a patient with sporadic bilateral retinoblastoma from the Mutant Human Gentic Cell Repository, Camden, NJ;

Lane 11, GM1231A retinoblastoma strain from the same patient as AG1484;

Lane 12, Molt-4, T-cell leukemia (Leavitt et al., J. Biol. Chem. (1980a) 255:4984-4987);

Lane 13, MG63 osteogenic sarcoma, ATCC No. CRL 1427;

Lane 14, Rat-2, a neoplastic rat cell line, (Leavitt et al., Nature (London) (1985) 316:840-842);

Lane 15, 3T3, a neoplastic mouse cell line (Leavitt et al. (1985), supra).

l-plastin mRNA and protein were not detected in the diploid, non-neoplastic human fibroblast strains KD and R17 but both strains exhibited t-plastin mRNA and protein (Gel B). Greater than 50 additional diploid human fibroblast strains derived from embryonic lung, skin, foreskin, and gingiva from normal individuals and from patients with various genetic diseases such as Huntington's disease, Bloom's syndrome, Ataxia telangiectasia, and retinoblastoma were examined. Without exception all diploid fibroblast strains exhibited expression of t-plastin in 2-D protein profiles, but not l-plastin. The two transformed fibroblast strains, HuT-12 and HuT-14, derived following mutagenesis of the KD cell culture (Leavitt et al. (1980a) supra), exhibited increasing levels of l-plastin polypeptide. The tumorigenic strain HuT-14 expressed more l-plastin mRNA and protein than the three tumorigenic HuT strains (HuT-11, -12, and -13). HuT-14T, an even more tumorigenic substrain of HuT-14 (Leavitt et al., Cell (1982) 28:259-2268, Leavitt et al., J. Mol. Biol. (1986) 6:2721-2726) was not elevated further in l-plastin expression.

Two cell lines derived from tumor tissue, the human fibrosarcoma cell line (HT1080) and lieomyosarcoma cell line (Sarcoma-2), also expressed l-plastin mRNA transcripts at levels comparable to the HuT strains. These two cell lines were previously shown to express levels of l-plastin protein comparable to the HuT strains (Goldstein et al. (1985a), supra). The osteogenic sarcoma cell line HOS synthesized a low level of l-plastin which is barely detectable in 2-D gels (Goldstein et al. (1985a), supra), but 1-plastin mRNA could not be detected in HOS cells by Northern blot analysis. A second osteogenic sarcoma, MG63, and a retinoblastoma tumor cell line GM 1231A, did not exhibit either the I-plastin mRNA transcript or the I-plastin polypeptide. All of the cell strains listed above expressed relatively constant levels of t-plastin mRNA transcripts and polypeptides except for the retinoblastoma cell line which exhibited no detectable plastin mRNA or protein at all.

I-Plastin expression in additional neoplastic human cell strains derived from ovarian carcinoma, endometrial carcinoma, choriocarcinoma tumors and in vitro transformed keratinccytes was observed. However, some established cell lines derived from an ovarian carcinoma, an adenocarcinoma of the cervix (Hela), a Wilm's tumor and a colon carcinoma do not appear to express I-plastin. In all 17 independent transformed (neoplastic) human cell lines derived from connective, epithelial and endothelial tissues, expressed I-plastin while 12 cell lines derived from other tumor sources did not exhibit I-plastin expression. Three retinoblastoma tumors which have deleted the I-plastin linked ret locus are included in these 12 I-plastin-negative cell lines. One of the retinoblastoma tumor cell lines, GM1231A, is distinguished from all the other cell types in that it did not express either I- or t-plastin.

## Example 3

### Expression of Plastin Isoforms in Human Leukocytes

The three lymphocyte cell lines CEM and Molt-4 (both T-cell leukemias), and AG1484, a transformed B-lymphoblast cell line derived from the same patient as the retinoblastoma GM1231A, synthesized high levels of I-plastin mRNA, but no detectable t-plastin mRNA. Leukemic cell lines such as CEM and normal leukocytes such as peripheral blood leukocytes (Goldstein et al. (1985b), supra) and cultured monocytes synthesize the highest levels of I-plastin protein (Goldstein et al. (1985b), supra), but no t-plastin protein. More than 20 different lymphoblastoid cell lines were examined, the promyelocytic cell line HL-60 (Anderson et al., Cancer Res. (1985) 45:4995), red blood cells, peripheral blood leukocytes (PBLs) from more than 20 individuals, and subfractions of PBLs including T-cells, NK cells, granulocytes, polymorphonuclear leukocytes, and monocytes (Goldstein et al. (1985b), supra). Without exception, all cells of hemopoietic origin except fully differentiated red blood cells exhibited abundant synthesis of I-plastin (Goldstein et al. (1985b), supra), and failed to express t-plastin.

## Example 5

### Plastin Genomic Sequences

The genomic representation of both the I- and the t-plastin isoforms was studied in six cell strains including retinoblastoma tumor cells and the B lymphoblast cell line derived from the same retinoblastoma patient. Genomic DNA was prepared by the method described by Maniatis et al., Cell (1978) 15:687-701. Ten microgram of each genomic DNA was digested with HindIII to completion, electrophoresed in a 0.7% agarose, and blotted onto nitrocellulose. An eight-fold degenerate 20 nucleotide probe described in Table 1B (Oligo Probe - Antisense) was designed from an I-plastin peptide sequence that exhibited a relatively low degeneracy. This oligonucleotide was synthesized using an ABI solid phase synthesizer. To further minimize degeneracy, preferred codon usage for valine and the weak base pair stabilizing characteristics of inosine (I) in the isoleucine codon (Ohtsuka et al., J. Biol. Chem. (1985) 260:2605-2608) were used. The oligonucleotide probe was end labeled with $^{32}$P and used to screen the HuT-14 cDNA library as previously described (Lin et al., supra) except that hybridization was performed at 42°C and the hybridized filters were washed at room-temperature with 2x SSC.

Hybridization and washing conditions were identical to those described for Table 1. Two identical genomic blots were hybridized with 32P-labeled cDNAs of P4 and P107, respectively. Each blot contained HindIII-digested genomic DNAs of KD (Lane 1), HuT-12 (Lane 2), HuT-14 (Lane 3), peripheral blood lymphocytes (Lane 4), B lymphoblast strain AG1484 (Lane 5), and retinoblastoma tumor cell strain GM1231A (Lane 6). Size markers were HindIII fragments of phage DNA. All human cell strains are described above.

The results indicated that the l- and t-plastin genes are located on separate DNA restriction fragments in the human genome, and there was no detectable difference in the restriction digest pattern of the t-plastin gene among all six human cell strains examined. There was also no detectable difference in the restriction digest pattern of the l-plastin gene between diploid human fibroblasts which do not synthesize l-plastin and transformed HuT-12 and HuT-14 human fibroblast strains which synthesize increasing levels of the l-plastin, respectively.

The genomic DNA of peripheral blood leukocytes derived from a healthy human donor had two HindIII fragments of 6.5 kb and 6.0 kb instead of the single band of 6.5 kb found in the other five genomic DNA samples. This individual's peripheral blood leukocytes synthesized the variant (polymorphic) form of the l-plastin (Goldstein et al. (1985b, supra) in addition to the normal charge species of l-plastin. The 6.0 kb fragment therefore may be indicative of this variant form of l-plastin.

The genomic DNA of the B lymphoblastoid cell line derived from the retinoblastoma patient exhibited approximately a 50% reduction in hybridization of l-plastin restriction fragments compared to the other five genomic DNA samples including the retinoblastoma tumor from the same patient. The reduced copy number of l-plastin genomic fragments apparent for this cell line was consistent with the observation that the l-plastin gene is linked to the retinoblastoma locus because one of the retinoblastoma alleles has been lost as a result of a large deletion around the retinoblastoma locus on chromosome 13. However, l-plastin genomic sequences did not appear to be reduced to the same extent in the genome of the retinoblastoma of the same patient, ruling out the possibility that the l-plastin gene was tightly linked to the retinoblastoma locus.

In conclusion, the profound degrees of activation or of inactivation observed for both the l-plastin and t-plastin genes in the set of cell strains represented in these six genomic DNA samples does not appear to be caused by gross deletions or rearrangements in the structure of these plastin genes.

## Summary of Experimental Results

## Significance of Plastin Isoforms

The plastin gene family that is described above encodes a truly novel set of at least two related but distinct proteins whose expression distinguished cells of solid tissue from hemopoietic or leukocyte cells. l-Plastin is a stable protein that is synthesized constitutively at a very high rate in a majority of subtypes of peripheral blood leukocytes and is one of the 10 or 20 most abundant proteins of these normal leukocyte cells. t-Plastin currently defines two equally abundant polypeptide species in fibroblasts, epithelial and endothelial cells that have the same molecular weight as l-plastin, but are slightly more basic.

The divergence of l- and t-plastin is striking because these two proteins, though clearly related, have diverged by 17 percent of their amino acids with the replacements scattered almost randomly throughout the 570 amino acid residues of the plastin sequence. Thirty three percent of these amino acid exchanges involve charged amino acid replacements with 7 short peptide domains exhibiting significantly greater divergence (residues 1-12 [50%] residues 18-30 [58%], residues 224-230 [57%], residues 258-275 [50%], residues 449-459 [50%], residues 487-496 [50%], and residues 512-523 [58%]). Both the 5′ and 3′ untranslated sequences of these two mRNAs were much more divergent than the sequences within the reading frames.

Finally, the induction of l-plastin expression in human fibroblasts does not appear to correlate with the activation of known oncogenes, since the HuT strains, which lack detectable "activated" oncogenes that will transform 3T3 cells (Cooper et al., Cancer Res. (1984) 44:1-10), and HT1080 cells, which have an activated N-ras oncogene (Paterson et al., Cell (1987) 51:803-812), abundantly express l-plastin.

The present invention provides novel methods of identifying hemopoietic cells of all types. The invention also provides methods and reagents for determining tissue cells which are in a neoplastic state. The presence of the plastin isoform indicative of the particular states is readily identified using either isoform-specific nucleotide probes or isoform-specific anti-plastin antibodies.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

14

**Claims**

1. A DNA fragment of fewer than about 50 kbp comprising a DNA sequence encoding human plastin and the 5' and 3' flanking non-coding regions.

2. The DNA sequence of Claim 1 wherein said plastin is leukocyte-plastin (l-plastin).

3. The DNA sequence of Claim 1 wherein said plastin is tissue-plastin (t-plastin).

4. The DNA fragment of Claim 1 wherein said fragment is a cDNA sequence of not more than about 5 kbp.

5. A probe of at least about 20 nt and not more than about 50 kbp comprising a nucleotide sequence including at least about 20 consecutive nt complementary to a DNA sequence encoding a plastin isoform or transcribed, non-coding regions of the plastin gene.

6. The probe of Claim 5 wherein said probe has less than about 60% homology with a DNA sequence encoding the other plastin isoform.

7. The probe of Claim 5 wherein said probe is complementary to a DNA sequence encoding the N-terminal 20 amino acids of said plastin isoform.

8. The probe of Claim 5 wherein said probe is a DNA sequence.

9. A peptide of less than about 100 amino acids comprising at least about 5 consecutive amino acids of the N-terminal 20 amino acids of l-plastin or t-plastin or sequences immunologically crossreactive therewith.

10. A peptide of less than about 100 amino acids comprising at least about 10 consecutive amino acids of:

    (a) MLDGDRNKDGKISFDEFVYI; or

    (b) MATGDLDQDGRISFDEFIKI.


11. An antibody composition which reacts with a plastin isoform and exhibits substantially no reaction with the other plastin isoform.

12. A method of determining whether a cell is neoplastic comprising:

determining the presence of l-plastin and t-plastin in said cell,

wherein a cell comprising both l-plastin and t-plastin is determined to be neoplastic.

13. The method of Claim 12 wherein said determining comprises detecting the presence of t-plastin mRNA and l-plastin mRNA in said cell.

14. The method of Claim 12 wherein said determining comprises detecting the presence of t-plastin and l-plastin proteins in said cell.

15. A method of determining whether a cell is a hemopoietic cell comprising:

determining whether said cell comprises t-plastin and l-plastin;

wherein said cell is determined to be hemopoietic when said cell comprises l-plastin but not t-plastin.

16. A method of determining whether a cell is neoplastic comprising:

(a) combining under hybridizing conditions RNA from said cell with a probe of at least about 20 nt and not more than about 50 kbp comprising a nucleotide sequence including at least about 20 consecutive nt complementary to a DNA sequence encoding t-plastin or adjacent transcribed non-coding regions; and

(b) combining under hybridizing conditions RNA from said cell with a probe of at least about 20 nt and not more than about 50 kbp comprising a nucleotide sequence including at least about 20 consecutive nt complementary to a DNA sequence encoding l-plastin or adjacent transcribed non-coding regions;

wherein a cell having RNA which hybridizes with both said probes is determined to be neoplastic.

17. A method of determining whether a cell is neoplastic comprising:

(a) contacting said cell with an antibody composition which reacts with t-plastin and exhibits substantially no reaction with l-plastin;

(b) contacting said cell with an antibody composition which reacts with l-plastin and exhibits substantially no reaction with t-plastin;

wherein a cell which reacts with both said antibody compositions is determined to be neoplastic.

18. The method of Claim 17 wherein each of said antibody compositions is labeled and said cell is stained by said antibody compositions.

19. A method of determining whether sample contains neoplastic cells comprising:

(a) contacting said cell-containing sample with an antibody composition which reacts with t-plastin and exhibits substantially no reaction with l-plastin;

(b) contacting a cell-containing sample containing with an antibody composition which reacts with l-plastin and exhibits substantially no reaction with t-plastin;

wherein said sample is determined to contain neoplastic cells when cells within said sample react with both said antibody compositions.